# FASCICULE DE BREVET EUROPEEN

(11) **EP 0 652 763 B1**
(45) Date de publication et mention de la délivrance du brevet: **07.01.1998**
(21) Numéro de dépôt: 93916015.6
(22) Date de dépôt: 20.07.1993
(51) Int. Cl.: A61K 35/80, A61K 7/48, C12P 1/00

(54) **EXTRAITS BRUTS D'ALGUES BLEUES, LEURS PROCEDES DE PREPARATION ET LEURS APPLICATIONS EN COSMETOLOGIE ET EN DERMATOLOGIE**
ROHEXTRAKTE DER BLAUALGE, HERSTELLUNGSMETHODE UND VERWENDUNG IN DER KOSMETOLOGIE UND DERMATOLOGIE
RAW EXTRACTS OF BLUE ALGAE, METHOD OF PREPARATION AND APPLICATIONS IN COSMETOLOGY AND DERMATOLOGY

(30) Priorité: 21.07.1992 FR 9209002
(43) Date de publication de la demande: 17.05.1995
(73) Titulaire: GENERALE DE PREVENTION ET DE LOISIRS, 03200 Vichy (FR)
(72) Inventeur: PEPIN, Denise, F-63400 Chamalières (FR); JEAN, Daniel, F-63270 Vic-le-Comte (FR)
(74) Mandataire: Peaucelle, Chantal
(86) Numéro de dépôt international: FR9300735
(87) Numéro de publication internationale: WO9402161

(56) Documents cités:
- BE-A- 693 094
- FR-A- 2 609 246
- CHEMICAL ABSTRACTS, Vol. 87, No. 20, November 1977, Columbus, Ohio, US, Abstract No. 157000u, "Spirulina Hydrolyzates For Cosmetic Packs", page 301; & JP,A,52 031 836, (YOSHIDA, RYUZO), 10 March 1977.
- CHEMICAL ABSTRACTS, Vol. 91, 1979, Columbus, Ohio, US, Abstract No. 37795t, NAKAZAWA, RIKUO, "Seaweed Extracts For Food", page 501; & JP,A,54 035 234, (NAKAZAWA, RIKUO), 15 March 1979.

## Description

La présente invention est relative à des extraits bruts d'algues bleues (cyanophycées), à leurs procédés de préparation et à leurs applications en cosmétologie et en dermatologie.

La peau est une barrière fragile, sujette à de nombreuses agressions.

De nombreux produits ont été proposés pour améliorer sa protection ou pour la traiter ; on peut notamment citer l'acide hyaluronique, l'élastine, le collagène ou l'ADN. Toutefois, ces produits ont l'inconvénient majeur d'avoir un effet superficiel et fugace, notamment en raison de l'absence de leur pénétration jusqu'au derme.

La Demanderesse a trouvé que des extraits bruts d'algues bleues présentent, de manière inattendue, des propriétés de protection et de restructuration de la peau en cosmétologie et en dermatologie.

La présente invention a pour objet des extraits bruts d'algues bleues, du type comprenant au moins une substance active choisie parmi les dérivés à noyau porphinique suivants : chlorophylles et carotène, caractérisés en ce qu'ils contiennent en outre au moins l'une des substances suivantes : chlorophyllides, chlorophyllines, phaéophytines et phaéophorbides, et en ce qu'il est susceptible d'être obtenu à partir d'une algue bleue, par décoction ou macération desdites algues, lyophilisées ou fraîches, à une température comprise entre 20° et 60°C, dans un solvant choisi dans le groupe constitué par les huiles minérales, les huiles végétales, les succédanés synthétiques d'huiles naturelles, les solvants organiques peu polaire et l'eau.

La présente invention a également pour objet des extraits bruts d'algues bleues, du tue comprenant au moins une substance active choisie parmi les dérivés à noyau porphinique suivants : chlorophylles et carotène, caractérisé en ce qu'il contient en outre au moins l'une des substances suivantes : chlorophylles, chlorophyllides, chlorophyllines, phaéophytines et phaéophorbides et en ce qu'il est susceptible d'être obtenu à partir de l'algue bleue du genre *Plectonema*, par décoction ou macération desdites algues, lyophilisées ou fraîches, à une température comprise entre 20° et 60°C, dans un solvant choisi dans le groupe constitué par les huiles minérales, les huiles végétales, les succédanés synthétiques d'huiles naturelles, les solvants organiques peu polaire, l'eau et les solvants miscibles à l'eau.

De manière avantageuse, il s'agit d'extraits bruts de l'algue bleue *Plectonema*.

La souche axénisée de *Plectonema* (élimination de tout contaminant bactérien) se présente sous forme de masses gélatineuses de couleur vert émeraude.

De manière générale, cette algue comprend :
. des glucides, sous forme libre et sous forme condensée, principalement du mannose et du glucose.
. des lipides : les lipides totaux représentent de 2 à 12 % du poids sec de l'algue, incluant notamment de l'alcool triterpénique, de la vitamine A et des acides gras dont les principaux participant à la composition de l'algue sont les suivants :
   - Acide palmitique: environ 50 % des acides gras totaux
   - Acide oléique: environ 15 % des acides gras totaux
   - C18:1ω7: environ 10 % des acides gras totaux
   - C18:2ω6: environ 4 % des acides gras totaux
   - Acide stéarique: environ 3 % des acides gras totaux
   - C16:1ω7: environ 1 % des acides gras totaux.
. des protides, qui occupent une place très importante dans la composition chimique de l'algue (de 20 à 70 % du poids sec).
. des acides aminés libres, qui représentent de 1 à 7 % du poids sec de l'algue, et essentiellement : alanine, phénylalanine, acide glutamique, valine et
. des pigments :
   - de structure porphinique : chlorophylles, chlorophyllides, phaéophytines, phaéophorbides,
   - de type caroténoïdique : β-carotène, zéaxanthine, autres caroténoïdes non identifiés et
   - de la phycocyanine : pigment bleu instable de nature protéique et caractéristique des cyanophycées.

De tels extraits, notamment obtenus à partir de cyanophycées du genre *Plectonema*, contiennent certaines substances à noyau porphinique, (voir la Demande Internationale PCT, déposée le même jour, au nom de la COMPAGNIE FERMIERE DE L'ETABLISSEMENT THERMAL DE VICHY, revendiquant une priorité française n° 92 09001, et concernant une famille de substances à noyau porphinique et une souche de *Plectonema sp*.), qui, de manière inattendue, sont actives sur la restructuration (notamment par leur effet sur la stimulation de la synthèse protéique des cellules) et la protection de la peau (notamment par leur effet anti-protéase), à la fois en cosmétologie (lutte contre les effets du vieillissement naturel de la peau, protection solaire, restructuration des peaux abîmées par les UV et d'autres agents agressifs et lutte contre les vergetures) et en dermatologie, dans certaines affections de la peau (inflammation due aux protéases, couperose, effets déstructurants dus aux corticoïdes, cicatrisation) et dans le traitement des brûlures.

De manière avantageuse, ledit extrait brut peut, en outre, être soumis à une filtration.

Selon un mode de réalisation avantageux desdits extraits bruts, ils sont susceptibles d'être obtenus en mettant en oeuvre des huiles végétales et minérales, choisies parmi l'huile de vaseline, l'huile de noyaux, l'huile de tournesol, l'huile d'amande douce, l'huile de sésame et l'huile d'arachide et des succédanés synthétiques d'huile naturelle, choisis parmi le myristate d'isopropyle, l'oléate de décyle et l'adipate de dibutyle.

Selon un autre mode de réalisation avantageux desdits extraits bruts, ils sont susceptibles d'être obtenus en mettant en oeuvre un solvant peu polaire, choisi parmi le chloroforme, le chlorure de méthylène, l'éther de pétrole ou l'hexane.

Selon une disposition avantageuse de ce mode de réalisation, ils sont susceptibles d'être obtenus par évaporation dudit solvant peu polaire, après ladite macération, pour l'obtention d'un extrait sec dont la coloration dépend du solvant utilisé.

De manière avantageuse, l'utilisation de tels solvants peu polaires permet d'obtenir des extraits secs liposolubles.

Selon un autre mode de réalisation desdits extraits, ils sont susceptibles d'être obtenus en mettant en oeuvre de l'eau, avantageusement associée à du chloroforme.

Selon encore un autre mode de réalisation desdits extraits, ils sont susceptibles d'être obtenus en mettant en oeuvre des solvants, miscibles à l'eau, choisis parmi l'éthanol, le méthanol et l'acétone.

De manière avantageuse, dans le cas d'utilisation de solvants aqueux, on obtient des extraits hydrosolubles.

Conformément à l'invention, lorsqu'ils sont susceptibles d'être obtenus par extraction à l'aide d'un solvant miscible à l'eau, on peut avantageusement évaporer le solvant et reprendre l'extrait obtenu dans l'eau.

De tels extraits trouvent application en cosmétologie et en dermatologie, sous la forme de compositions comprenant entre 0,1 et 3 % (en poids) d'extrait brut conforme à l'invention, et sont éventuellement associés à d'autres substances actives et/ou excipients convenables.

En cosmétologie, ces compositions trouvent notamment application dans la lutte contre le vieillissement naturel de la peau, dans la restructuration des peaux abîmées par les UV et les autres agents agressifs, dans le traitement des vergetures et pour la protection solaire.

En dermatologie, ces compositions trouvent application pour le traitement de la couperose, dans la lutte contre les effets déstructurants des corticoïdes, dans la lutte contre les effets des protéases, dans l'inflammation, dans la cicatrisation et dans le traitement des brûlures.

La présente invention a également pour objet un procédé de préparation desdits extraits bruts, caractérisé en ce qu'il comprend les étapes suivantes :
- décoction ou macération d'une algue bleue, lyophilisée ou fraîche, à une température comprise entre 20° et 60°C, dans un solvant choisi dans le groupe constitué par les huiles minérales, les huiles végétales, les succédanés synthétiques d'huiles naturelles, les solvants organiques peu polaire, l'eau et les solvants miscibles à l'eau (lorsque l'algue bleue est du genre *Plectonema*) et, si nécessaire
- filtration de l'extrait obtenu.

Selon un mode de réalisation avantageux dudit procédé, l'algue bleue est du genre *Plectonema*.

La présente invention a également pour objet une composition cosmétique, caractérisée en ce qu'elle renferme au moins un extrait brut d'algue bleue, contenant au moins une substance active choisie parmi les dérivés à noyau porphinique suivants : chlorophylles, chlorophyllides, chlorophyllines, phaéophytines et phaéophorbides et susceptible d'être obtenu à partir d'une algue bleue, par décoction ou macération desdites algues, lyophilisées ou fraîches, à une température comprise entre 20° et 60°C, dans un solvant choisi dans le groupe constitué par les huiles minérales, les huiles végétales, les succédanés synthétiques d'huiles naturelles, les solvants organiques peu polaire, l'eau et les solvants miscibles à l'eau.

Selon un mode de réalisation avantageux de ladite composition, ledit extrait brut contient, en outre, au moins un pigment caroténoïque choisi parmi le β-carotène et la zéaxanthine.

Selon un autre mode de réalisation avantageux de ladite composition, l'algue bleue est du genre *Plectonema*.

Selon encore un autre mode de réalisation avantageux de ladite composition, ledit extrait brut est soumis à une filtration.

Selon un autre mode de réalisation avantageux de ladite composition, ledit extrait brut est susceptible d'être obtenu en mettant en oeuvre des huiles végétales et minérales, choisies parmi l'huile de vaseline, l'huile de noyaux, l'huile de tournesol, l'huile d'amande douce, l'huile de sésame et l'huile d'arachide et des succédanés synthétiques d'huile naturelle, choisis parmi le myristate d'isopropyle, l'oléate de décyle et l'adipate de dibutyle.

Selon encore un autre mode de réalisation avantageux de ladite composition, ledit extrait brut est susceptible d'être obtenu en mettant en oeuvre un solvant peu polaire, choisi parmi le chloroforme, le chlorure de méthylène, l'éther de pétrole ou l'hexane.

Selon une disposition avantageuse de ce mode de réalisation, ledit extrait brut est susceptible d'être obtenu en évaporant le solvant peu polaire, après ladite macération, pour l'obtention d'un extrait sec dont la coloration dépend du solvant utilisé.

Selon un autre mode de réalisation avantageux de ladite composition, ledit extrait brut est susceptible d'être obtenu en mettant en oeuvre de l'eau, associée à du chloroforme.

Selon encore un autre mode de réalisation avantageux de ladite composition, ledit extrait brut est susceptible d'être obtenu en mettant en oeuvre des solvants, miscibles à l'eau, choisis parmi l'éthanol, le méthanol et l'acétone.

Selon une disposition avantageuse de ce mode de réalisation, lorsque ledit extrait brut est susceptible d'être obtenu par extraction à l'aide d'un solvant miscible à l'eau, on peut avantageusement évaporer le solvant et reprendre l'extrait obtenu dans l'eau.

Selon encore un autre mode de réalisation avantageux de ladite composition, ledit extrait brut est à une concentration comprise entre 0,1 et 3 % (en poids).

La présente invention a également pour objet une méthode de traitement esthétique de l'Homme pour protéger et restructurer la peau, comprenant l'administration d'une quantité appropriée d'un extrait brut conforme à l'invention, éventuellement associé à au moins un véhicule acceptable.

Outre les dispositions qui précèdent, l'invention comprend encore d'autres dispositions, qui ressortiront de la description qui va suivre, qui se réfère à des exemples de mise en oeuvre du procédé objet de la présente invention.

Il doit être bien entendu, toutefois, que ces exemples sont donnés uniquement à titre d'illustration de l'objet de l'invention, dont ils ne constituent en aucune manière une limitation.

### EXEMPLE 1 : Préparation d'extraits liposolubles.

### 1) Extrait liposoluble n° 1 :

Il est préparé exclusivement à partir d'algues lyophilisées, car la présence d'eau est nuisible à une bonne extraction.

### * Principe :

Extraction d'une phase liposoluble par macération de l'algue lyophilisée, donc sèche dans une huile minérale ou végétale, dans un succédané synthétique d'huile naturelle tel que le myristate d'isopropyle, l'oléate de décyle ou l'adipate de dibutyle.

Cette macération peut-être réalisée à une température comprise entre 20°C et 60°C.

### * Exemple :

un kilo d'algues lyophilisées est placé dans 20 litres d'huile de noyaux et le tout est maintenu à 40°C pendant 48 heures.

La solution huileuse obtenue est tamisée sur un tamis en acier inox de 100 µm afin d'éliminer la plus grande quantité d'algue, puis filtrée à 40°C sur une toile de porosité 10 µm. On obtient 19,7 litres d'une solution limpide de couleur jaune verdâtre utilisable dans des formules cosmétiques.

Cet extrait est dénommé extrait liposoluble n° 1.

### 2) Extrait liposoluble n° 2 :

### * Principe :

L'algue lyophilisée est extraite par décoction ou macération dans un solvant organique peu polaire tel que le chloroforme, le chlorure de méthylène, l'éther de pétrole ou l'hexane.

Le solvant est ensuite évaporé pour laisser la place à un extrait de coloration et de composition dépendant du solvant employé.

Le chloroforme va, par exemple, extraire les pigments de type chlorophylliens alors que l'hexane va extraire exclusivement les pigments de type caroténoidique.

### * Exemple :

Un kilo d'algues lyophilisées sont soumises à trois décoctions successives à reflux pendant une heure dans du chloroforme.

Les solutions chloroformiques sont filtrées sur papier, réunies, puis évaporées à sec sous pression réduite.

On obtient 124 g d'une pâte vert foncé à odeur caractéristique d'algue.

Cet extrait, soumis à l'analyse contient les pigments porphiniques énoncés plus haut suivants : chlorophylles, chlorophyllides, phaéophytines, phaéophorbides.

Il contient aussi tous les pigments caroténoïdiques présents dans l'algue.

### EXEMPLE 2 : Préparation d'extraits hydrosolubles.

### 1) Extrait hydrosoluble n° 1 :

### * Principe :

Les extraits hydrosolubles sont préparés par macération à froid ou traitement à chaud des algues fraîches ou lyophilisées, par de l'eau distillée. On ajoute une faible proportion de chloroforme dans l'eau pour éviter les fermentations en cours d'extraction. On peut aussi extraire les algues d'abord par un solvant miscible à l'eau tels que l'éthanol, le méthanol ou l'acétone, puis évaporer les solvants sous pression réduite et reprendre l'extrait ainsi obtenu par de l'eau distillée.

### * Exemples :

Cinq kilos d'algues fraîches sont placés dans 20 litres d'eau distillée à laquelle on a préalablement ajouté 10 ml de chloroforme.

On soumet à une agitation permanente pendant 24 heures. On tamise la solution aqueuse sur un tamis en acier inox de 100 µm et on filtre ensuite la solution sur une toile de porosité 10 µm. La solution aqueuse obtenue est ensuite lyophilisée. On obtient 67 g de lyophilisat.

Cet extrait contient des polysaccharides, des sucres libres, des sels minéraux, des acides aminés et des protéines mais très peu de pigments.

### 2) Extrait hydrosoluble n° 2 :

Un kilo d'algues lyophilisées sont décoctés trois fois de suite par de l'éthanol à 50 % à reflux pendant une heure. Les décoctats sont filtrés à chaud sur papier, puis réunis et évaporés à sec sous pression réduite. On obtient 164 g d'extrait sec. L'extrait sec ainsi obtenu est repris par un litre d'eau distillée à 60°C sous agitation constante pendant une heure. On filtre sur papier à 60^{°}C, puis on lyophilise la solution aqueuse. On obtient alors 27 g de lyophilisat.

Cet extrait contient peu de protéines, des polysaccharides, des sucres libres, des sels minéraux et des pigments de type chlorophyllides.

Ces extraits peuvent notamment être obtenus à partir de l'algue *Plectonema*, issue du griffon de la source Boussange de Vichy et notamment à partir de la souche *Plectonema*, dénommée VY1, déposée le 29 mai 1991, sous le n° I-1101.

Ces différents extraits, qui contiennent les différentes substances actives précitées à des concentrations différentes, peuvent être utilisés seuls ou en mélange selon l'effet recherché.

### EXEMPLE 3 : Effets des extraits obtenus sur la physiologie de la peau.

### * Accroissement de la résistance du collagène aux effets de la collagénase :

### - Mise en évidence :

On répartit dans quatre boîtes de Pétri de 8 cm de diamètre, 20 ml d'une solution à 1 % d'agarose dans du tampon Tris pH 8,8 contenant en suspension 20 mg de collagène fibreux insoluble et 0, 0,1 0,3 et 0,5 ml d'une solution à 5 % de produit à tester (extrait brut) selon les boîtes, soient des concentrations de 0, 0,025 %, 0,075 %, 0,125 %. Comme la plupart des produits à tester ne sont pas solubles dans le tampon, on part d'une solution mère dans du diméthylsulfoxyde (DMSO). Cette répartition se fait à 45^{°}C.

Après solidification de l'agarose, on perfore 5 puits de 3 mm de diamètre dans le gel grâce à un emporte pièce. Dans ces cinq puits, on place 50 µl d'une solution de collagénase pancréatique dans du tampon Tris pH 8,8 à une concentration de : 10 mg/ml (3800 U/ml).

Les boîtes de Pétri sont incubées 15 heures à 37°C, puis le gel est coloré par une solution à 0,25 % d'acide picrique à 0,25 % pendant 3 min, puis une solution de Rouge Sirius à 0,1 % pendant 5 min. L'excès de coloration est éliminé par lavage d'eau.

### - Résultats :

| Produit testé | 0,025 % | 0,05 % | 0,075 % |
|---|---|---|---|
| Extrait liposoluble 1 | 0 % | 0 % | 2 % |
| Extrait liposoluble 2 | 2 % | 22 % | 32 % |
| Extrait hydrosoluble 1 | 0 % | 0 % | 3 % |
| Extrait hydrosoluble 2 | 6 % | 17 % | 47 % |

On évalue l'action enzymatique en mesurant le diamètre des zones d'hydrolyse du collagène fibreux.

On constate un effet marqué pour l'extrait liposoluble 2 et l'extrait hydrosoluble 2 ; cette différence peut s'expliquer par le fait que, selon le mode d'extraction, on extrait plus ou moins de substances actives.

### * Effet d'inhibition de la collagénase :

### - Mise en évidence :

On répartit dans quatre boîtes de Pétri de 8 cm de diamètre, 20 ml d'une solution à 1 % d'agarose dans du tampon Tris pH 8,8, contenant en suspension 20 mg de collagène fibreux insoluble. Cette répartition se fait à 45°C.

Après solidification de l'agarose, on perfore 5 puits de 3 mm de diamètre dans le gel grâce à un emporte pièce. Dans ces cinq puits, on place 25 µl d'une de collagénase pancréatique dans du tampon Tris pH 8,8 à des concentrations de : 10 mg/ml (3800 U/ml) et 25 µl d'une solution de la substance à doser dans le même tampon à une concentration de 4 %, 3 %, 2 %, 1 % et 0 %. Comme la plupart des produits à tester ne sont pas solubles dans le tampon, on part d'une solution mère dans du diméthylsulfoxyde.

Les boîtes de Pétri sont incubées 15 heures à 37°C, puis le gel est coloré par une solution à 0,25 % d'acide picrique à 0,25 % pendant 3 min, puis une solution de Rouge Sirius à 0,1 % pendant 5 min. L'excès de coloration est éliminé par lavage à l'eau.

### - Résultats :

| Produit testé | 1 % | 2 % | 3 % | 4 % |
|---|---|---|---|---|
| Extrait liposoluble 1 | 0 % | 0 % | 1 % | 1 % |
| Extrait liposoluble 2 | 0 % | 7 % | 11 % | 14 % |
| Extrait hydrosoluble 1 | 0 % | 0 % | 1 % | 1 % |
| Extrait hydrosoluble 2 | 2 % | 4 % | 8 % | 12 % |

On constate également un effet marqué pour l'extrait liposoluble 2 et l'extrait hydrosoluble 2, pour les mêmes raisons que pour le test précédent.

### * Inhibition de l'élastase pancréatique :

### - Mise en évidence :

L'activité de l'élastase pancréatique est déterminée sur un substrat synthétique. On constate que les produits décrits ci-dessus ont un effet sur cette enzyme alors que le substrat est un dérivé de peptide de faible poids moléculaire : le N succinyl N ala-ala-ala p nitroanilide.

Solution substrat : solution du substrat ci-dessus à 1 % dans du tampon phosphate pH 7,6.

Solution enzymatique : élastase pancréatique à 1000 U/ml dans le tampon pH 7,6.

Solvant : DMSO à 50 % dans de l'eau distillée.

Solution à tester : extrait brut en solution dans le solvant à 0,1 %.

Dans un premier tube à essai (blanc), on place :
- 2,2 ml: de tampon pH 7,6
- 0,3 ml: de solution enzymatique
- 0,5 ml: de solvant.

Dans un deuxième tube à essai (essai), on place :
- 1 ml: de solution substrat
- 1,2 ml: de tampon pH 7,6
- 0,3 ml: de solution enzymatique
- 0,5 ml: de solution à tester.

Dans un troisième tube à essai, on place :
- 1 ml: de solution substrat
- 1,2 ml: de tampon pH 7,6
- 0,3 ml: de solution enzymatique
- 0,5 ml: de solvant.

On mesure la D.O. de la solution essai contre la solution blanc à 410 nm. On note la valeur toutes les 30 secondes pendant 30 minutes. On réalise la même mesure pour la solution témoin.

L'activité de l'enzyme est définie par la vitesse d'augmentation de D.O. du témoin.

L'activité de l'inhibiteur (extrait brut) se définit comme une diminution relative de l'activité de l'enzyme.

### - Résultats :

| Produit testé | |
|---|---|
| Extrait liposoluble 1 | 0 % |
| Extrait liposoluble 2 | 22 % |
| Extrait hydrosoluble 1 | 8 % |
| Extrait hydrosoluble 2 | 66 % |

On constate un effet marqué pour l'extrait liposoluble 2 et l'extrait hydrosoluble 2 ; les effets sont plus modestes pour les autres extraits. Cette différence s'explique par le fait que l'extrait brut peut contenir plus ou moins de substances actives, selon la manière dont il est extrait, comme précisé ci-dessus.

### * Effet des extraits bruts sur la synthèse des protéines totales par des fibroblastes en culture :

### - Mise en évidence :

Une étude de cytotoxicité est réalisée au préalable pour déterminer les concentrations maximales de test des différents extraits.

Cellules utilisées : fibroblastes humains MRC5, au 28ème passage.

Les cellules sont ensemencées sur une plaque à 96 puits, à raison de 2.10⁵ cellules par puits environ, et mises à cultiver dans du milieu minimum de Eagle (MEM) additionné de 1 % de glutamine 200 mM, 1 % de solution de vitamines, 100 U/ml de pénicilline, 100 U/ml de streptomycine et 2 % de sérum de veau foetal.

La solution à tester (extrait brut) est ajoutée à raison de 100 µl par puits à la concentration de 50 µg/ml.

Les cellules sont incubées à 37°C sous 5 % d'atmosphère de CO₂. On prélève le milieu de culture après 48, 72, 96 et 120 heures de culture.

Les protéines totales synthétisées sont déterminées après coloration des surnageants par le bleu de Coomassie et mesure de la D.O. à 595 nm. L'augmentation de la synthèse protéique est mesurée par rapport au témoin (solvant de la substance à tester).

### - Résultats :

| Produit testé | |
|---|---|
| Extrait liposoluble 1 | 0 % |
| Extrait liposoluble 2 | 8 % |
| Extrait hydrosoluble 1 | 0 % |
| Extrait hydrosoluble 2 | 16 % |

### * Effet antiradicalaire :

Deux techniques peuvent être utilisées pour mettre en évidence l'effet antiradicalaire *in vitro* des extraits bruts conformes à l'invention.

### - Mise en évidence par la méthode enzymatique :

- Substrat :: EDTA : 0,05 g
Xanthine : 0,0125 g
Cytochrome c : 0,02 g
Tampon phosphate pH 7,5 : 200 ml.
- Enzyme :: xanthine oxydase à 1 % dans le tampon.
- Solvant :: DMSO à 50 % dans de l'eau distillée.

Composition antiradicalaire (extrait conforme à l'invention) : 0,5 % dans le solvant.
- Solution blanc :: substrat 2 ml
tampon pH 7,5 : 0,5 ml
solvant : 0,5 ml
- Solution témoin :: substrat : 2 ml
solution enzymatique : 0,5 ml
solvant : 0,5 ml.
- Solution essai :: substrat : 2 ml
solution enzymatique : 0,5 ml
composition antiradicalaire : 0,5 ml.

On mesure la D.O. de la solution témoin contre la solution blanc à 550 nm toutes les 30 secondes, pendant 3 min.

Puis on mesure la D.O. de la solution essai contre la solution blanc toutes les 30 secondes pendant 30 min.

On détermine l'effet antiradicalaire comme le rapport des deux vitesses mesurées.

### - Résultats :

| Produit testé | |
|---|---|
| Extrait liposoluble 1 | 10 % |
| Extrait liposoluble 2 | 57 % |
| Extrait hydrosoluble 1 | 22 % |
| Extrait hydrosoluble 2 | 59 % |

On constate ici, que les résultats sont plus homogènes, ce qui montre à la fois l'intérêt des différents extraits et la présence de substances actives différentes, en tant qu'antiradicalaires, par rapport aux substances à effet antiprotéasique général.

### - Mise en évidence par la méthode chimique :

Réactif : 1,1-diphényl-2-picrylhydrazyle : 2,5 mg dans 100 ml de méthanol.

Solvant des extraits bruts à activité antiradicalaire : méthanol.

Composition antiradicalaire : 2 % dans du méthanol.

Ce réactif est un radical libre stable pouvant être dégradé par des substances antiradicalaires.

On réalise un blanc pour le témoin et un blanc pour l'essai :
- Blanc témoin: : tampon phosphate pH 7 : 1,5 ml
méthanol : 3 ml
- Témoin :: solution réactif : 3 ml
tampon phosphate pH 7 : 1,5 ml
- Blanc essai :: tampon phosphate pH 7 : 1,35 ml
méthanol : 3,15 ml
- Essai :: solution réactif : 3 ml
tampon phosphate pH 7 : 1,35 ml
solution antiradicalaire : 0,15 ml.

On agite à 25°C pendant 5 min. Puis on extrait par 4 ml de toluène pendant 30 secondes. On filtre sur un papier séparateur de phases et on lit la D.O. de la solution de toluène à 519 nm contre la solution de toluène obtenue pour le blanc correspondant. On calcule le pourcentage de réactif disparu.

### - Résultats :

| Produit testé | |
|---|---|
| Extrait liposoluble 1 | 21 % |
| Extrait liposoluble 2 | 62 % |
| Extrait hydrosoluble 1 | 41 % |
| Extrait hydrosoluble 2 | 72 % |

On constate ici également, que les résultats sont plus homogènes, ce qui montre la présence de principes actifs différents, en tant qu'antiradicalaires, par rapport aux effets antiprotéasiques.

### EXEMPLE 4 : Formulations cosméto-dermatologiques comprenant des extraits bruts conformes à l'invention.

Pour chaque formule, la concentration globale en extrait brut est comprise entre 0,1 et 3 %.

### * Crème :

| | |
|---|---|
| - Mélange de glycérides partiels, d'alcool gras, d'ester cireux et d'alcools gras éthoxylés | 10 |
| - Myristate d'isopropyle | 8 |
| - Huile de vaseline fluide | 3 |
| - Mélange de cyclométhicone et diméthicone | 3 |
| - Glycérine | 10 |
| Mélange de polyacrylamide, d'alcool gras éthoxylé et isoparaffine | 1 |
| - Mélange de Nipa esters (28 %) dans du phénoxyéthanol | 0,5 |
| - Parfum | 0,2 |
| - Soude à 1N | 0,6 |
| - Extrait liposoluble 2 | 2 |
| - Eau désionisée Q.S.P. | 100 |

### * Lait :

| | |
|---|---|
| - Glycéryl stéarate | 8 |
| - Monostéarate de glycérine de polyoxyéthylène | 3 |
| - Myristate d'isopropyle | 8 |
| - Stéarate d'isooctyle | 2 |
| - 2-octyl dodécanol | 5 |
| - Mélange de cyclométhicone et diméthicone | 3 |
| - Glycérine | 5 |
| - Mélange de Nipa esters (28 %) dans du phénoxyéthanol | 0,5 |
| - Parfum | 0,2 |
| - Soude à 1N | 0,5 |
| - Extrait liposoluble 1 | 1,5 |
| - Eau désionisée Q.S.P. | 100 |

### * Lotion :

| | |
|---|---|
| - 2-hydroxyalcoxylate d'alcool gras | 2 |
| - Polyolester d'acide gras | 2 |
| - Glycérine | 3 |
| - Alcool éthylique à 96 % | 20 |
| - Hydroxyéthylcellulose | 0,6 |
| - Parfum | 0,3 |
| - Extrait hydrosoluble 1 | 1 |
| - Eau désionisée Q.S.P. | 100 |

### * Gel :

| | |
|---|---|
| - Acide polyacrylique réticulé | 0,5 |
| - 2-hydroxyalcoxylate d'alcool gras | 2 |
| - Polyolester d'acide gras | 2 |
| - Glycérine | 2 |
| - Lessive de soude à 35 % | 0,43 |
| - Mélange de Nipa esters (28 %) dans du phénoxyéthanol | 0,5 |
| - Parfum en conformité avec les normes IFRA-RIFM | 0,2 |
| - Extrait hydrosoluble 2 | 5 |
| - Eau désionisée Q.S.P. | 100 |

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée de la présente invention.

## Revendications

1. Composition cosmétique, caractérisée en ce qu'elle renferme au moins un extrait brut d'algue bleue, contenant au moins une substance active choisie parmi les dérivés à noyau porphinique suivants : chlorophylles, chlorophyllides, chlorophyllines, phaéophytines et phaéophorbides et susceptible d'être obtenu à partir d'une algue bleue, par décoction ou macération desdites algues, lyophilisées ou fraîches, à une température comprise entre 20° et 60°C, dans un solvant choisi dans le groupe constitué par les huiles minérales, les huiles végétales, les succédanés synthétiques d'huiles naturelles, les solvants organiques peu polaire, l'eau et les solvants miscibles à l'eau.

2. Composition cosmétique selon la revendication 1, caractérisée en ce que ledit extrait brut contient, en outre, au moins un pigment caroténoïque choisi parmi le β-carotène et la zéaxanthine.

3. Composition cosmétique selon la revendication 1 ou la revendication 2, caractérisée ce que l'algue bleue est du genre *Plectonema*.

4. Composition cosmétique selon la revendication 1, caractérisée en ce que ledit extrait brut est soumis à une filtration.

5. Composition cosmétique selon l'une quelconque des revendications 1 à 4, caractérisée en ce que ledit extrait brut est susceptible d'être obtenu en mettant en oeuvre des huiles végétales et minérales, choisies parmi l'huile de vaseline, l'huile de noyaux, l'huile de tournesol, l'huile d'amande douce, l'huile de sésame et l'huile d'arachide et des succédanés synthétiques d'huile naturelle, choisis parmi le myristate d'isopropyle, l'oléate de décyle et l'adipate de dibutyle.

6. Composition cosmétique selon l'une quelconque des revendications 1 à 4, caractérisée en ce ledit extrait brut est susceptible d'être obtenu en mettant en oeuvre un solvant peu polaire, choisi parmi le chloroforme, le chlorure de méthylène, l'éther de pétrole ou l'hexane.

7. Composition cosmétique selon la revendication 6, caractérisée en ce que ledit extrait brut est susceptible d'être obtenu en évaporant le solvant peu polaire, après ladite macération, pour l'obtention d'un extrait sec dont la coloration dépend du solvant utilisé.

8. Composition cosmétique selon l'une quelconque des revendications 1 à 4, caractérisée en ce que ledit extrait brut est susceptible d'être obtenu en mettant en oeuvre de l'eau, associée à du chloroforme.

9. Composition cosmétique selon l'une quelconque des revendications 1 à 4, caractérisée en ce que ledit extrait brut est susceptible d'être obtenu en mettant en oeuvre des solvants, miscibles à l'eau, choisis parmi l'éthanol, le méthanol et l'acétone.

10. Composition cosmétique selon la revendication 9, caractérisée en ce que, lorsque ledit extrait brut est susceptible d'être obtenu par extraction à l'aide d'un solvant miscible à l'eau, on peut avantageusement évaporer le solvant et reprendre l'extrait obtenu dans l'eau.

11. Composition cosmétique selon l'une quelconque des revendications 1 à 10, caractérisée en ce que ledit extrait brut est à une concentration comprise entre 0,1 et 3 % (en poids).

12. Extrait brut d'algues bleues, du type comprenant au moins une substance active choisie parmi les dérivés à noyau porphinique suivants : chlorophylles et carotène, caractérisé en ce qu'il contient en outre au moins l'une des substances suivantes : chlorophyllides, chlorophyllines, phaéophytines et phaéophorbides, et en ce qu'il est susceptible d'être obtenu à partir d'une algue bleue, par décoction ou macération desdites algues, lyophilisées ou fraîches, à une température comprise entre 20° et 60°C, dans un solvant choisi dans le groupe constitué par les huiles minérales, les huiles végétales, les succédanés synthétiques d'huiles naturelles, les solvants organiques peu polaire et l'eau.

13. Extrait brut d'algues bleues, du type comprenant au moins une substance active choisie parmi les dérivés à noyau porphinique suivants : chlorophylles et carotène, caractérisé en ce qu'il contient en outre au moins l'une des substances suivantes : chlorophylles, chlorophyllides, chlorophyllines, phaéophytines et phaéophorbides et en ce qu'il est susceptible d'être obtenu à partir de l'algue bleue du genre *Plectonema*, par décoction ou macération desdites algues, lyophilisées ou fraîches, à une température comprise entre 20° et 60°C, dans un solvant choisi dans le groupe constitué par les huiles minérales, les huiles végétales, les succédanés synthétiques d'huiles naturelles, les solvants organiques peu polaire, l'eau et les solvants miscibles à l'eau.

14. Extrait brut d'algues bleues selon la revendication 12 ou la revendication 13, caractérisé en ce qu'il contient, en outre, au moins un pigment caroténoïque choisi parmi la zéaxanthine.

15. Extrait brut selon la revendication 12 ou la revendication 13, caractérisé en ce qu'il est soumis à une filtration.

16. Extrait brut selon l'une quelconque des revendications 12 à 15, caractérisé en ce qu'il est susceptible d'être obtenu en mettant en oeuvre des huiles végétales et minérales, choisies parmi l'huile de vaseline, l'huile de noyaux, l'huile de tournesol, l'huile d'amande douce, l'huile de sésame et l'huile d'arachide et des succédanés synthétiques d'huile naturelle, choisis parmi le myristate d'isopropyle, l'oléate de décyle et l'adipate de dibutyle.

17. Extrait brut selon l'une quelconque des revendications 12 à 15, caractérisé en ce qu'il est susceptible d'être obtenu en mettant en oeuvre un solvant peu polaire, choisi parmi le chloroforme, le chlorure de méthylène, l'éther de pétrole ou l'hexane.

18. Extrait brut selon la revendication 17, caractérisé en ce qu'il est susceptible d'être obtenu en évaporant le solvant peu polaire, après ladite macération, pour l'obtention d'un extrait sec dont la coloration dépend du solvant utilisé.

19. Extrait brut selon l'une quelconque des revendications 12 à 15, caractérisé en ce qu'il est susceptible d'être obtenu en mettant en oeuvre de l'eau, associée à du chloroforme.

20. Extrait brut selon la revendication 13, caractérisé en ce qu'il est susceptible d'être obtenu en mettant en oeuvre des solvants, miscibles à l'eau, choisis parmi l'éthanol, le méthanol et l'acétone.

21. Extrait brut selon la revendication 20, caractérisé en ce que, lorsque il est susceptible d'être obtenu par extraction à l'aide d'un solvant miscible à l'eau, on peut avantageusement évaporer le solvant et reprendre l'extrait obtenu dans l'eau.

22. Méthode de traitement esthétique de l'Homme pour protéger et restructurer la peau, comprenant l'administration d'une quantité appropriée d'un extrait brut selon l'une quelconque des revendications 12 à 21, éventuellement associé à au moins un véhicule acceptable.

23. Composition pharmaceutique, caractérisée en ce qu'elle comprend au moins un extrait brut selon l'une quelconque des revendications 12 à 21, éventuellement associé à d'autres substances actives et/ou à au moins un excipient convenable.

24. Composition selon la revendication 23, caractérisée en ce que ledit extrait brut est à une concentration comprise entre 0,1 et 3 % (en poids).

25. Composition pharmaceutique, utile en tant qu'agent de protection et de restructuration de la peau, caractérisée en ce qu'elle renferme un extrait brut selon l'une quelconque des revendications 12 à 21.

26. Procédé de préparation d'un extrait brut selon la revendication 12, caractérisé en ce qu'il comprend les étapes suivantes :
- décoction ou macération d'une algue bleue, lyophilisée ou fraîche, à une température comprise entre 20° et 60°C, dans un solvant choisi dans le groupe constitué par les huiles minérales, les huiles végétales, les succédanés synthétiques d'huiles naturelles, les solvants organiques peu polaire et l'eau et, si nécessaire
- filtration de l'extrait obtenu.

27. Procédé de préparation d'un extrait brut selon la revendication 13, caractérisé en ce qu'il comprend les étapes suivantes :
- décoction ou macération d'une algue bleue, lyophilisée ou fraîche, à une température comprise entre 20° et 60°C, dans un solvant choisi dans le groupe constitué par les huiles minérales, les huiles végétales, les succédanés synthétiques d'huiles naturelles, les solvants organiques peu polaire, l'eau et les solvants miscibles à l'eau et, si nécessaire
- filtration de l'extrait obtenu.

## Claims

1. A cosmetic composition, characterized in that it contains at least one raw extract of blue algae containing at least one active substance chosen from the following derivatives with a porphin nucleus: chlorophylls, chlorophyllides, chlorophyllins, phaeophytins and phaeophorbides, and may be obtained from a blue algae by decoction or maceration of said algae, freeze-dried or fresh, at a temperature between 20 and 60C, in a solvent chosen from the group consisting of mineral oils, vegetable oils, synthetic substitutes of natural oils, relatively non polar organic solvents, water, and solvents which are miscible with water.

2. A cosmetic composition according to Claim 1, characterized in that said raw extract contains, in addition, at least one carotenoid pigment chosen from β-carotene and zeaxanthin.

3. A cosmetic composition according to Claim 1 or Claim 2, characterized in that the blue algae is of the *Plectonema* genus.

4. A cosmetic composition according to Claim 1, characterized in that said raw extract undergoes filtration.

5. A cosmetic composition according to any one of Claims 1 to 4, characterized in that said raw extract may be obtained using vegetable and mineral oils chosen from liquid paraffin, kernel oil, sunflower oil, sweet almond oil, sesame oil and arachis oil and synthetic substitutes of natural oil, chosen from isopropyl myristate, decyl oleate and dibutyl adipate.

6. A cosmetic composition according to any one of Claims 1 to 4, characterized in that said raw extract may be obtained using a relatively non polar solvent chosen from chloroform, methylene chloride, petroleum ether or hexane.

7. A cosmetic composition according to Claim 6, characterized in that said raw extract may be obtained by evaporating the relatively non polar solvent, after said maceration, in order to obtain a dry extract the colour of which depends on the solvent used.

8. A cosmetic composition according to any one of Claims 1 to 4, characterized in that said raw extract may be obtained using water combined with chloroform.

9. A cosmetic composition according to any one of Claims 1 to 4, characterized in that said raw extract may be obtained using solvents which are miscible with water, chosen from ethanol, methanol and acetone.

10. A cosmetic composition according to Claim 9, characterized in that, if said raw extract is capable of being obtained by extraction with the aid of a solvent which is miscible with water, the solvent may be advantageously evaporated and the extract obtained taken up in water.

11. A cosmetic composition according to any one of Claims 1 to 10, characterized in that said raw extract is in a concentration between 0.1 and 3% (by weight).

12. A raw extract of blue algae, of the type containing at least one active substance chosen from the following derivatives with a porphin nucleus: chlorophylls and carotene, characterized in that it also contains at least one of the following substances: chlorophyllides, chlorophyllins, phaeophytins and phaeophorbides, and in that it may be obtained from a blue algae by decoction or maceration of said algae, freeze-dried or fresh, at a temperature between 20 and 60C in a solvent chosen from the group consisting of mineral oils, vegetable oils, synthetic substitutes of natural oils, relatively non polar organic solvents and water.

13. Raw extract of blue algae of the type containing at least one active substance chosen from the following derivatives with a porphin nucleus: chlorophylls and carotene, characterized in that it also contains at least one of the following substances: chlorophylls, chlorophyllides, chlorophyllins, phaeophytins and phaeophorbides, and in that it may be obtained from a blue algae of the *Plectonema* genus by decoction or maceration of said algae, freeze-dried or fresh, at a temperature between 20 and 60C in a solvent chosen from the group consisting of mineral oils, vegetable oils, synthetic substitutes of natural oils, relatively non polar organic solvents, water, and solvents that are miscible with water.

14. A raw extract of blue algae according to Claim 12 or Claim 13, characterized in that it also contains at least one carotenoid pigment chosen from zeaxanthin.

15. A raw extract according to Claim 12 or Claim 13, characterized in that it undergoes filtration.

16. A raw extract according to any one of Claims 12 to 15, characterized in that it may be obtained using vegetable and mineral oils chosen from liquid paraffin, kernel oil, sunflower oil, sweet almond oil, sesame oil and arachis oil and synthetic substitutes of natural oil, chosen from isopropyl myristate, decyl oleate and dibutyl adipate.

17. A raw extract according to any one of Claims 12 to 15, characterized in that it may be obtained using a relatively non polar solvent chosen from chloroform, methylene chloride, petroleum ether or hexane.

18. A raw extract according to Claim 17, characterized in that it may be obtained by evaporating the relatively non polar solvent, after said maceration, in order to obtain a dry extract the colour of which depends on the solvent used.

19. A raw extract according to any one of Claims 12 to 15, characterized in that it may be obtained using water combined with chloroform.

20. A raw extract according to Claim 13, characterized in that it may be obtained using solvents which are miscible with water, chosen from ethanol, methanol and acetone.

21. A raw extract according to Claim 20, characterized in that, if it is capable of being obtained by extraction with the aid of a solvent which is miscible with water, the solvent may be advantageously evaporated and the extract obtained taken up in water.

22. A method for the aesthetic treatment of Man for protecting and restructuring the skin, comprising the administration of an appropriate quantity of a raw extract according to any one of Claims 12 to 21, optionally combined with at least one acceptable vehicle.

23. A pharmaceutical composition, characterized in that it comprises at least one raw extract according to any one of Claims 12 to 21, optionally combined with other active substances and/or at least one suitable excipient.

24. A composition according to Claim 23, characterized in that said raw extract is in a concentration between 0.1 and 3% (by weight).

25. A pharmaceutical composition, useful as an agent for protecting and restructuring the skin, characterized in that it contains a raw extract according to any one of Claims 12 to 21.

26. A process for the preparation of a raw extract according to Claim 12, characterized in that it comprises the following steps:
- decoction or maceration of a blue algae, freeze-dried or fresh, at a temperature between 20 and 60C, in a solvent chosen from the group consisting of mineral oils, vegetable oils, synthetic substitutes of natural oils, relatively non polar organic solvents and water and, if necessary,
- filtration of the extract obtained.

27. A process for the preparation of a raw extract according to Claim 13, characterized in that it comprises the following steps:
- decoction or maceration of a blue algae, freeze-dried or fresh, at a temperature between 20 and 60C, in a solvent chosen from the group consisting of mineral oils, vegetable oils, synthetic substitutes of natural oils, relatively non polar organic solvents, water, and solvents which are miscible with water and, if necessary,
- filtration of the extract obtained.

## Patentansprüche

1. Kosmetische Zusammensetzung, dadurch gekennzeichnet, daß sie wenigstens einen Blaualgenextrakt einschließt, der wenigstens einen Wirkstoff enthält, der unter den nachfolgenden Verbindungen mit einem Porphyrinkern ausgewählt ist: Chlorophylle, Chlorophyllide, Chlorophylline, Phäophytine und Phäophorbide und der aus einer Blaualge durch Abkochen oder Mazerieren der gefriergetrockneten oder frischen Algen bei einer Temperatur zwischen 20°C und 60°C in einem Lösungsmittel, ausgewählt aus der aus Mineralölen, Pflanzenölen, synthetischen Ersatzstoffen für natürliche Öle, schwach polaren organischen Lösungsmitteln, Wasser und den mit Wasser mischbaren Lösungsmitteln bestehenden Gruppe, erhältlich ist.

2. Kosmetische Zusammensetzung nach Anspruch 1, dadurch gekennzeichnet, daß der Rohextrakt zusätzlich wenigstens ein unter β-Carotin und Zeaxanthin ausgewähltes Carotinpigment enthält.

3. Kosmetische Zusammensetzung nach Anspruch 1 oder Anspruch 2, dadurch gekennzeichnet, daß die Blaualge von der Gattung Plectonema ist.

4. Kosmetische Zusammensetzung nach Anspruch 1, dadurch gekennzeichnet, daß der Rohextrakt einer Filtration unterworfen wird.

5. Kosmetische Zusammensetzung nach einem der Ansprüche 1 bis 4, dadurch gekennzeichnet, daß der Rohextrakt durch Anwendung von pflanzlichen und mineralischen Ölen, ausgewählt unter Vaselineöl, Nußöl, Sonnenblumenöl, Süßmandelöl, Sesamöl und Erdnußöl, und von synthetischen Ersatzstoffen des natürlichen Öls, ausgewählt unter Isopropylmyristat, Decyloleat und Dibutyladipat, erhältlich ist.

6. Kosmetische Zusammensetzung nach einem der Ansprüche 1 bis 4, dadurch gekennzeichnet, daß der Rohextrakt durch Anwendung eines schwach polaren Lösungsmittels, ausgewählt unter Chloroform, Methylenchlorid, Petrolether oder Hexan, erhältlich ist.

7. Kosmetische Zusammensetzung nach Anspruch 6, dadurch gekennzeichnet, daß der Rohextrakt durch Verdampfen des schwach polaren Lösungsmittels nach dem Mazerieren zur Gewinnung eines Trockenextraktes, dessen Färbung vom verwendeten Lösungsmittel abhängt, erhältlich ist.

8. Kosmetische Zusammensetzung nach einem der Ansprüche 1 bis 4, dadurch gekennzeichnet, daß der Rohextrakt durch Anwendung von Wasser, in Verbindung mit Chloroform, erhältlich ist.

9. Kosmetische Zusammensetzung nach einem der Ansprüche 1 bis 4, dadurch gekennzeichnet, daß der Rohextrakt durch Anwendung von mit Wasser mischbaren Lösungsmitteln, ausgewählt unter Ethanol, Methanol und Aceton, erhältlich ist.

10. Kosmetische Zusammensetzung nach Anspruch 9, dadurch gekennzeichnet, daß dann, wenn der Rohextrakt durch Extraktion mit Hilfe eines mit Wasser mischbaren Lösungsmittels erhältlich ist, das Lösungsmittel vorteilhaft verdampft werden kann und der erhaltene Extrakt im Wasser aufgenommen werden kann.

11. Kosmetische Zusammensetzung nach einem der Ansprüche 1 bis 10, dadurch gekennzeichnet, daß der Rohextrakt in einer Konzentration von 0,1 bis 3 Gew.-% vorliegt.

12. Blaualgenrohextrakt vom Typ, der wenigstens einen Wirkstoff, ausgewählt unter den folgenden Verbindungen mit einem Porphyrinkern, umfaßt: Chlorophylle und Carotin, dadurch gekennzeichnet, daß er zusätzlich wenigstens eine der folgenden Substanzen enthält: Chlorophyllide, Chlorophylline, Phäophytine und Phäophorbide, und daß er aus einer Blaualge durch Abkochen oder Mazerieren der gefriergetrockneten oder frischen Algen bei einer Temperatur zwischen 20°C und 60°C in einem Lösungsmittel erhältlich ist, das aus der Gruppe gewählt wird, die aus mineralischen Ölen, pflanzlichen Ölen, synthetischen Ersatzstoffen von natürlichen Ölen, schwach polaren organischen Lösungsmitteln und Wasser besteht.

13. Blaualgenrohextrakt, vom Typ, der wenigstens einen Wirkstoff, ausgewählt unter den folgenden Verbindungen mit einem Porphyrinkern, enthält: Chlorophylle und Carotin, dadurch gekennzeichnet, daß er zusätzlich wenigstens eine der folgenden Substanzen enthält: Chlorophylle, Chlorophyllide, Chlorophylline, Phäophytine und Phäophorbide, und daß er aus der Blaualge der Gattung Plectonema durch Abkochen oder Mazerieren der gefriergetrockneten oder frischen Algen bei einer Temperatur zwischen 20°C und 60°C in einem Lösungsmittel erhältlich ist, das aus der Gruppe gewählt wird, die aus mineralischen Ölen, pflanzlichen Ölen, synthetischen Ersatzstoffen von natürlichen Ölen, schwach polaren organischen Lösungsmitteln, Wasser und den mit Wasser mischbaren Lösungsmitteln besteht.

14. Blaualgenrohextrakt nach Anspruch 12 oder Anspruch 13, dadurch gekennzeichnet, daß er zusätzlich wenigstens ein unter Zeaxanthin ausgewähltes Carotinpigment enthält.

15. Rohextrakt nach Anspruch 12 oder Anspruch 13, dadurch gekennzeichnet, daß er einer Filtration unterworfen wird.

16. Rohextrakt nach einem der Ansprüche 12 bis 15, dadurch gekennzeichnet, daß er durch Anwendung von pflanzlichen und mineralischen Ölen, ausgewählt unter Vaselineöl, Nußöl, Sonnenblumenöl, Süßmandelöl, Sesamöl und Erdnußöl, und von synthetischen Ersatzstoffen für natürliches Öl, ausgewählt unter Isopropylmyristat, Decyloleat und Dibutyladipat, erhältlich ist.

17. Rohextrakt nach einem der Ansprüche 12 bis 15, dadurch gekennzeichnet, daß er unter Anwendung eines schwach polaren Lösungsmittels, ausgewählt unter Chloroform, Methylenchlorid, Petrolether oder Hexan, erhältlich ist.

18. Rohextrakt nach Anspruch 17, dadurch gekennzeichnet, daß er durch Verdampfen des schwach polaren Lösungsmittels nach dem Mazerieren zur Gewinnung eines Trockenextraktes, dessen Färbung vom verwendeten Lösungsmittel abhängt, erhältlich ist.

19. Rohextrakt nach einem der Ansprüche 12 bis 15, dadurch gekennzeichnet, daß er durch Anwendung von Wasser, in Verbindung mit Chloroform, erhältlich ist.

20. Rohextrakt nach Anspruch 13, dadurch gekennzeichnet, daß er durch Anwendung von mit Wasser mischbaren Lösungsmitteln, ausgewählt unter Ethanol, Methanol und Aceton, erhältlich ist.

21. Rohextrakt nach Anspruch 20, dadurch gekennzeichnet, daß dann, wenn der Extrakt durch Extraktion mit Hilfe eines mit Wasser mischbaren Lösungsmittels erhältlich ist, in vorteilhafter Weise das Lösungsmittel verdampft und der erhaltene Extrakt im Wasser wiederaufgenommen werden kann.

22. Verfahren zur ästhetischen Behandlung des Menschen zum Schützen und Restrukturieren der Haut, umfassend die Verabreichung einer geeigneten Menge eines Rohextraktes nach einem der Ansprüche 12 bis 21, gegebenenfalls in Verbindung mit wenigstens einem annehmbaren Träger.

23. Pharmazeutische Zusammensetzung, dadurch gekennzeichnet` daß sie wenigstens einen Rohextrakt nach einem der Ansprüche 12 bis 21 enthält, gegebenenfalls in Verbindung mit anderen Wirkstoffen und/oder mit wenigstens einem geeignetem Exzipienten.

24. Zusammensetzung nach Anspruch 23, dadurch gekennzeichnet, daß der Rohextrakt in einer Konzentration von 0,1 bis 3 Gew.-% enthalten ist.

25. Pharmazeutische Zusammensetzung, die gleichzeitig als Mittel zum Schützen und zum Restrukturieren der Haut nützlich ist, dadurch gekennzeichnet, daß sie einen Rohextrakt nach einem der Ansprüche 12 bis 21 einschließt.

26. Verfahren zur Herstellung eines Rohextraktes nach Anspruch 12, dadurch gekennzeichnet, daß es die folgenden Stufen umfaßt:
- Abkochen oder Mazerieren einer gefriergetrockneten oder frischen Blaualge bei einer Temperatur von 20°C bis 60°C in einem Lösungsmittel, das aus der Gruppe ausgewählt ist, die aus mineralischen Ölen, pflanzlichen Ölen, synthetischen Ersatzstoffen für natürliche Öle, schwach polaren organischen Lösungsmitteln und Wasser besteht, und, soferne erforderlich,
- Filtrieren des erhaltenen Extraktes.

27. Verfahren zur Herstellung eines Rohextraktes nach Anspruch 13, dadurch gekennzeichnet, daß es die folgenden Stufen umfaßt:
- Abkochen oder Mazerieren einer gefriergetrockneten oder frischen Blaualge bei einer Temperatur von 20 bis 60°C in einem Lösungsmittel, das aus der Gruppe ausgewählt ist, die aus mineralischen Ölen, Pflanzenölen, synthetischen Ersatzstoffen für natürliche Öle, schwach polaren organischen Lösungsmitteln, Wasser und mit Wasser mischbaren Lösungsmitteln besteht, und, soferne erforderlich,
- Filtrieren des erhaltenen Extraktes.
